# EUROPEAN PATENT SPECIFICATION

(11) **EP 0 527 752 B1**
(45) Date of publication and mention of the grant of the patent: **12.05.1999**
(21) Application number: 91906598.7
(22) Date of filing: 28.03.1991
(51) Int. Cl.: C12N 1/19, C12N 15/81, C12N 15/31

(54) **EXPRESSION OF BORDETELLA PERTACTIN ANTIGENS IN PICHIA**
EXPRESSION VON BORDETELLA ANTIGENEN IN PICHIA
EXPRESSION DES ANTIGENS DE BORDETELLA DANS LA LEVURE PICHIA

(30) Priority: 02.04.1990 GB 9007416
(43) Date of publication of application: 24.02.1993
(60) Divisional application: 98115190.5
(73) Proprietor: Medeva Pharma Limited, Leatherhead, Surrey KT22 7PQ (GB)
(72) Inventor: CLARE, Jeffrey, John Langley Court, Kent BR3 3BS (GB); ROMANOS, Michael, Anthony Langley Court, Kent BR3 3BS (GB)
(74) Representative: Woods, Geoffrey Corlett
(86) International application number: GB9100487
(87) International publication number: WO9115571

(56) References cited:
- Proc. Natl. Acad. Sci., volume 86, 1989, (Washington, DC, US), I.G. Charles et al.: "Molecular cloning and characterization of protective outer membrane protein P.69 from Bordetella pertussis", pages 3554-3558
- Biotechnology, volume 5, no. 5, May 1987, (New York, NY, US), J.M. Clegg et al.: "High-level expression and efficient assembly of hepatitis B surface antigen in the methylotrophic yeast, Pichia Pastoris", pages 479-485
- Yeast, volume 6, spec. issue, S428, 1990, 15th International Conference on Yeast genetics and Molecular Biology, M.A. Romanos et al.: "Expression of the bordatella pertussis (whooping cough) P,69 antigen in saccharomyces and picchia", page 2
- Belsham et al. Eur. J. Biochem. 156: 413-21 (1986)
- Janowicz et al. Gene 20: 347-58 (1982)
- Jabber et al. PNAS 82: 2019-23 (1985)

## Description

The present invention relates to the expression of heterologous protein in yeast and more particularly to the production of Bordetella pertactin antigens in Pichia.

Bordetella pertussis causes whooping cough, an acute respiratory disease which is serious and debilitating in humans, children being particularly susceptible. The organism is responsible for approximately 1 million deaths each year, although this is being to some extent controlled in the developed countries by large scale immunisation programmes. It has been found that immunisation against B.pertussis is very effective at preventing the disease, and that failure to vaccinate does lead to increased incidence of the disease. In practically all areas, immunisation is effected using a whole cell B.pertussis vaccine which has been found to be relatively effective in preventing the disease and infant mortality.

However, public acceptance of whole cell vaccines has decreased due to side-effects and controversy over rare neurological complications attributed to such vaccine preparations. Consequently, researchers have been looking for safer, effective, acellular vaccines consisting of purified Bordetella antigens.

Surface antigen of B.pertussis is known to elicit a humoral and a cellular immune response in humans. It is disclosed as ACAP in EP-A-162639 and is now known as P.69 (I.G. Charles et al. Proc.Natl.Acad.Sci. USA, vol. 80, 3554-3558 (1989)). It is likely to be an important component of any future acellular vaccine against B.pertussis infections.

B.parapertussis and B.bronchiseptica are closely related to the B.pertussis organism. B.parapertussis is also responsible for outbreaks of whooping cough in man (Zeuler et al. J.pediatr. 9:493-497 (1946); B.bronchiseptica is known to cause respiratory diseases in animals, particularly atrophic rhinitis in pigs (Harris and Switzer Am.J. Vet. Res. 29 777-785 (1968)).

B.parapertussis and B.bronchiseptica appear to present antigens related to B.pertussis P.69, with molecular masses of 70 and 68kDa respectively. These Bordetella antigens, which are referred to hereinafter as 'pertactin antigens', are known to bind to the BB05 antibody but appear to have different immunogenic properties (I.G. Charles et al. Proc. Natl. Acad. Sci. USA Vol. 80 3554-3558 (1989)). Only small amounts of pertactin antigen can be isolated from cultures of Bordetella organism. It is preferable for the production of antigens on a commercial scale, to be able to produce large quantities.

E.coli is known as a host organism for the manufacture of heterologous proteins such as antigens, in quantity, but has certain drawbacks since it contains toxic pyrogenic factors (lipopolysaccharides from the cell wall) which must be rigorously excluded from the final product. The ease with which these factors may be excluded will depend on the method of purification. However, it would be advantageous to eliminate the possibility of contamination altogether simply by using a non-toxic organism as the host, such as yeast.

When baker's yeast, Saccharomyces cerevisiae, is used as the host organism, poor expression levels of heterologous protein are frequently obtained. (Kingsman, et al., Biotechnology & Genet. Engin. Reviews. Vol. 3 377-416, 1985). Use of the yeast Pichia pastoris as a host for the expression of heterologous protein is also known (EP-A-0180899 and EP-A-0263311). However, expression of membrane proteins in yeast is generally problematic since these proteins can interact with yeast cell membranes causing toxic effects to the yeast cell and reduced product yields. Examples of such difficulties have been described and include the expression of polyoma virus middle-T antigen (Belsham, et al. Eur. J. Biochem. 156 413-421, 1986); expression of the bacterial membrane protein OmpA (Janowitz, *et al.* Gene 20, 347-358, 1982); and expression of influenza virus haemagglutinin (Jabbar, *et al.,* Proc. Natl. Acad. Sci. USA 82, 2019-2023, 1985).

The present inventors have found a means of producing good levels of expression of the *Bordetella* pertactin antigens by culturing *Pichia* transformants containing multiple copies of the DNA encoding a pertactin antigen or an antigenic fragment thereof.

Accordingly, the present invention provides a *Pichia* microorganism transformed with multiple copies of DNA which encodes a pertactin antigen or an antigenic fragment thereof, the said multiple copies being integrated into the *Pichia* chromosomal DNA.

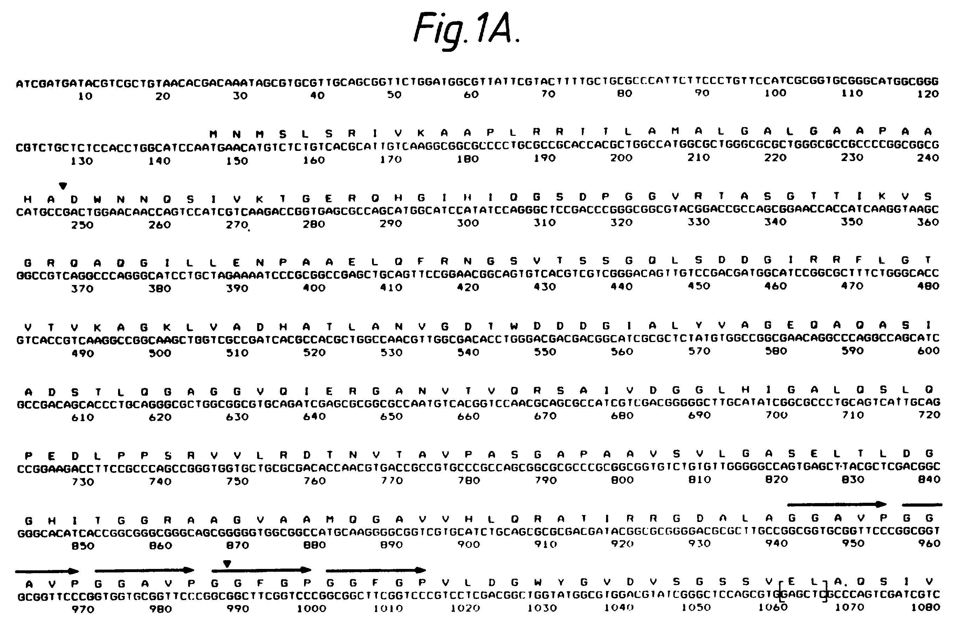

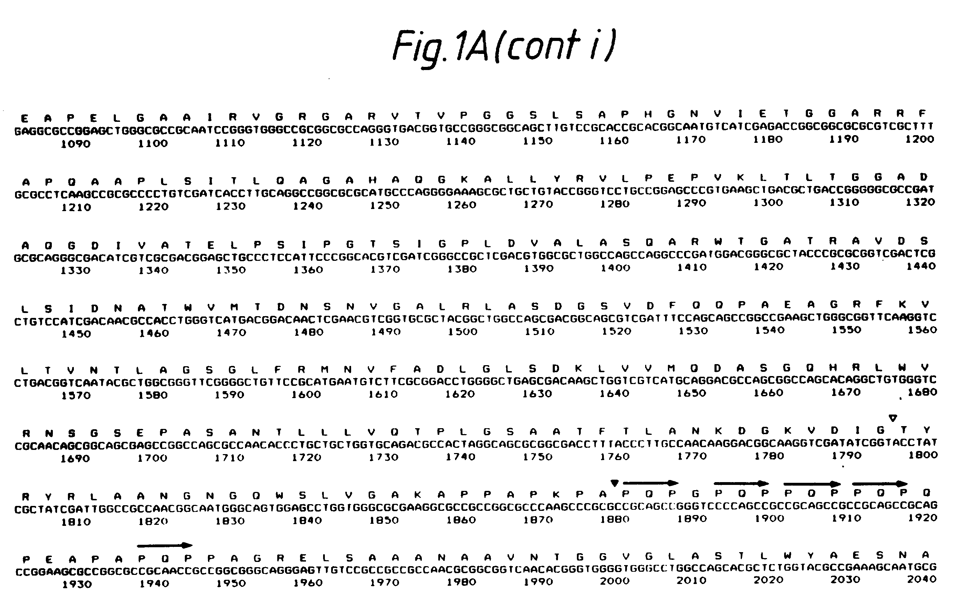

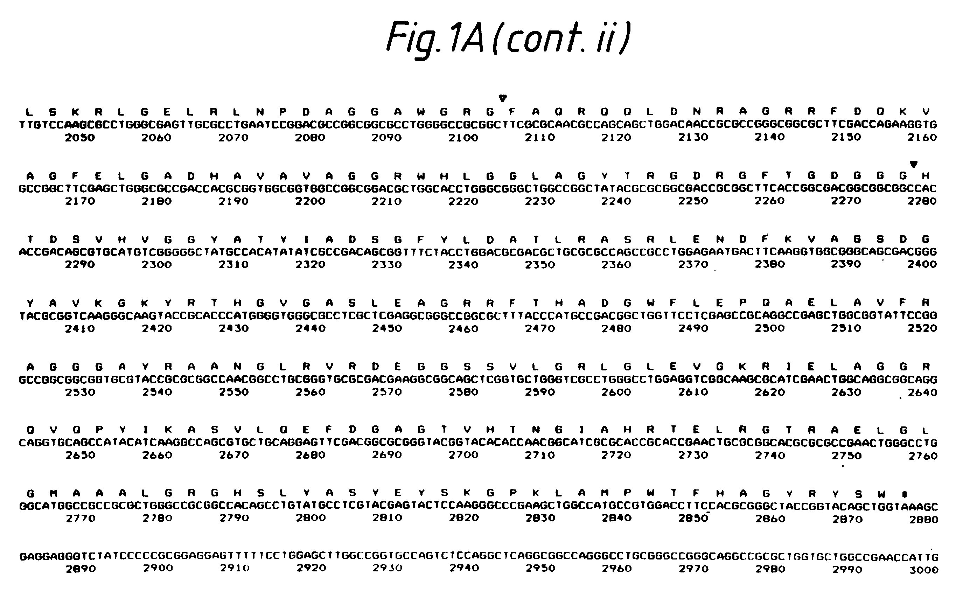

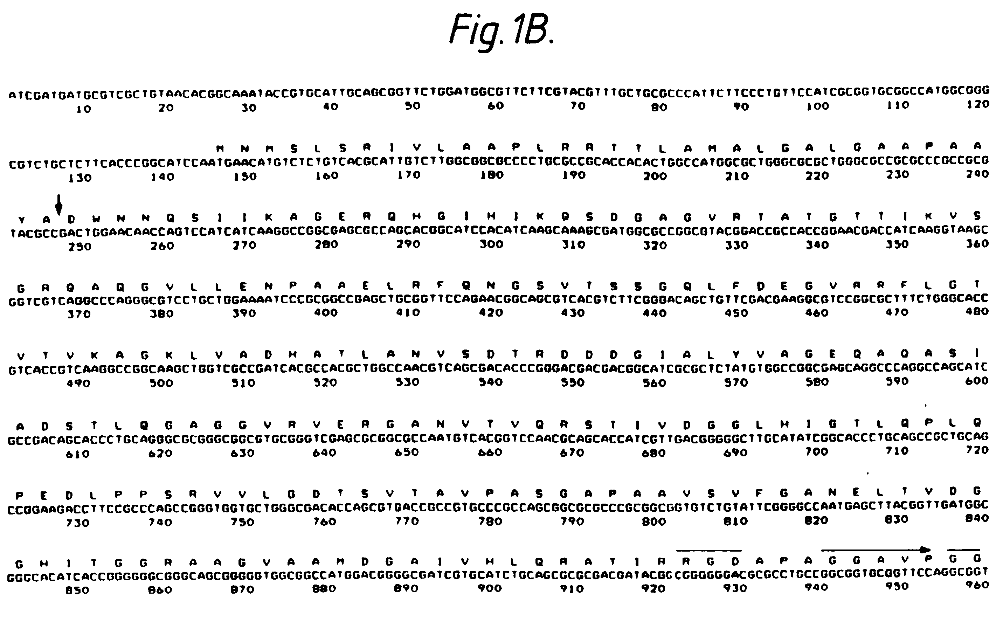

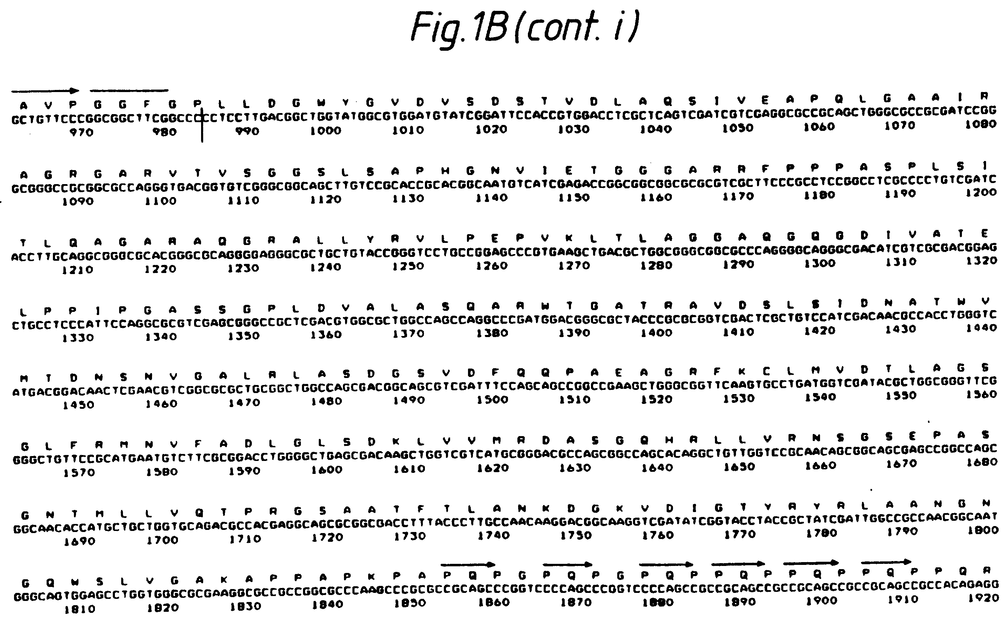

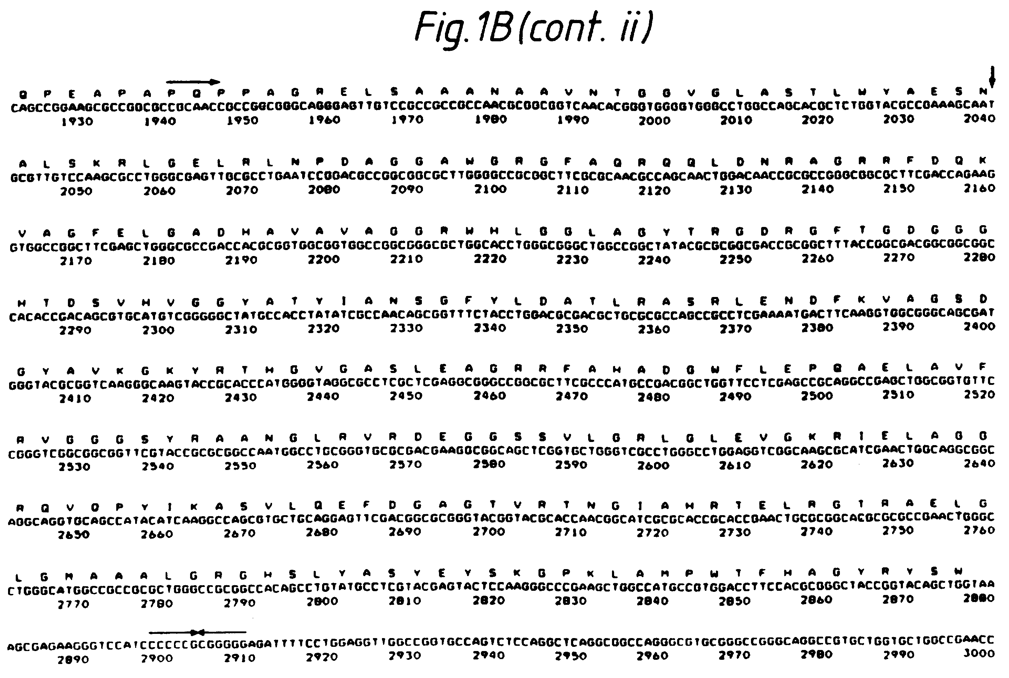

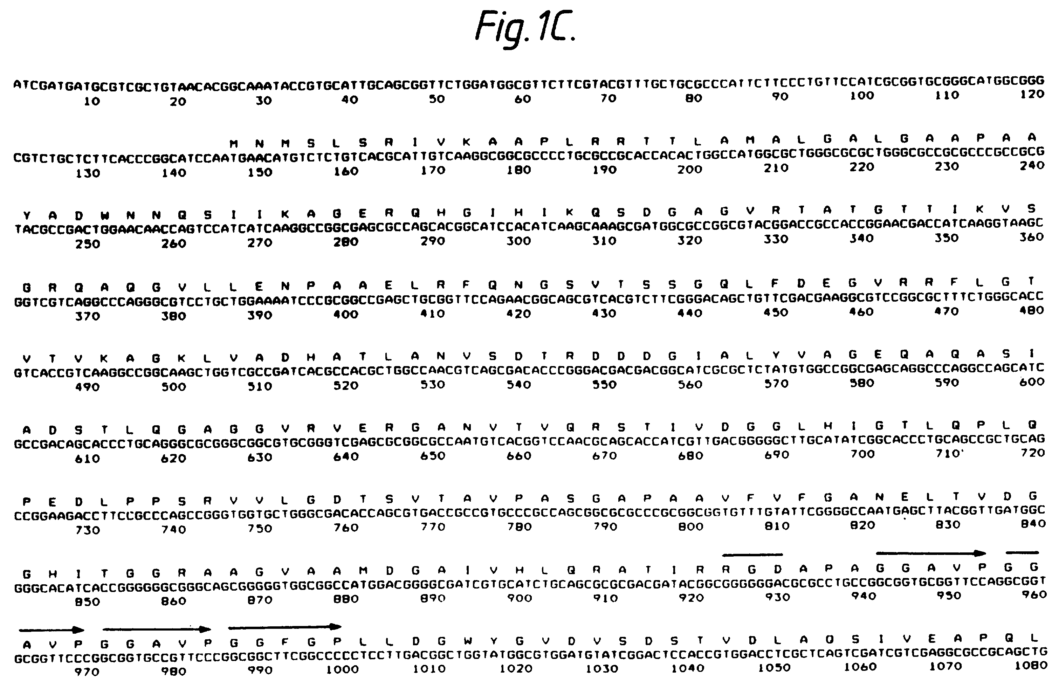

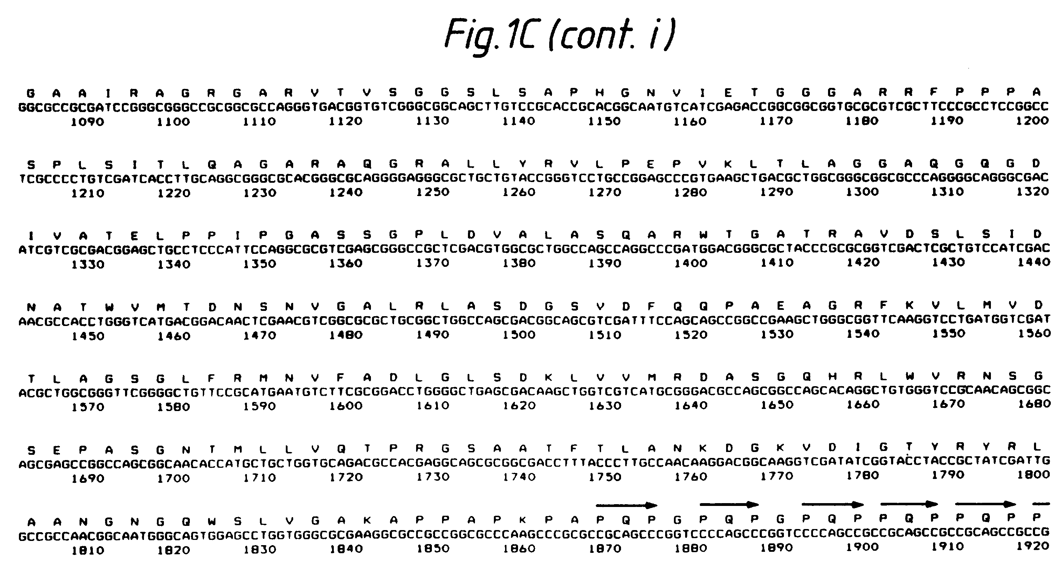

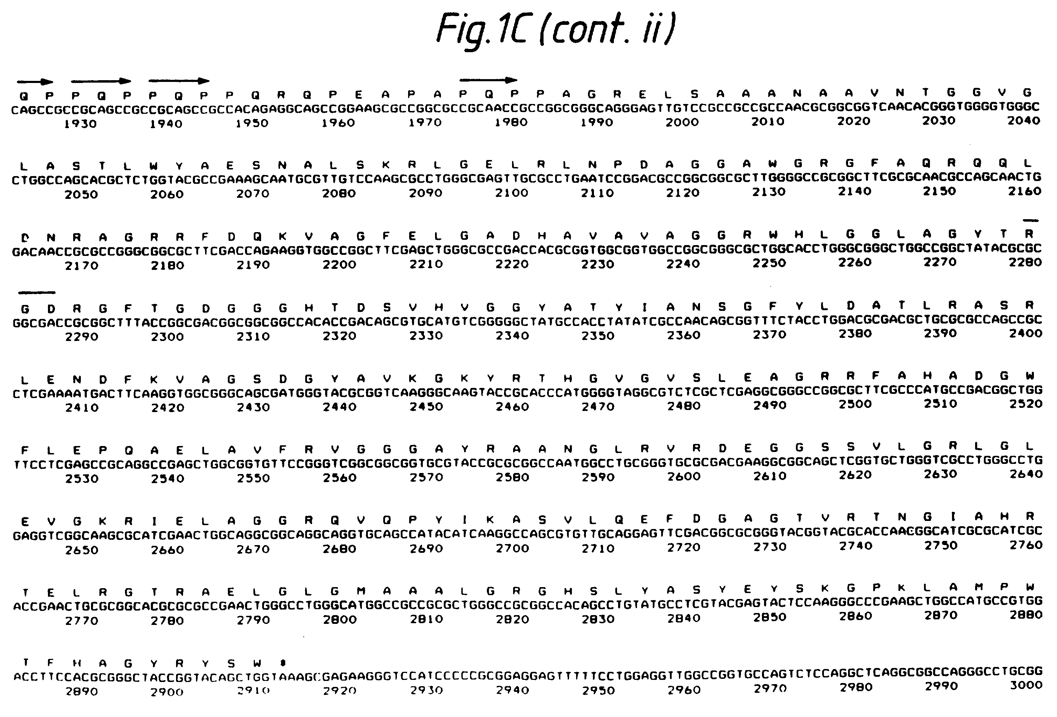

The *Pichia* microorganism can thus be transformed with DNA for the expression of a pertactin antigen whose amino acid sequence is at least 95% homologous with that set forth in Figure 1A, 1B or 1C, or an antigenic fragment thereof. It is preferable if the amino acid sequence described above is at least 98% homologous with that set forth in Figure 1A, 1B or 1C, or an antigenic fragment thereof.

A pertactin antigen from *B.pertussis* includes the antigen whose amino acid sequence is at least 95% homologous with, but is preferably substantially the same as, that set out in Figure 1A. This antigen is denoted P69. A *pertactin* antigen from *B.bronchiseptica* includes the antigen whose amino acid sequence is at least 95% homologous with, but is preferably substantially the same as, that set out in Figure 1B. This antigen is denoted P68. A pertactin antigen from *B.parapertussis* includes the antigen whose amino acid sequence is at least 95% homologous with, but is preferably substantially the same as, that set out in Figure 1C. This antigen is denoted P70.

The DNA for the expression of a pertactin antigen may encode a larger precursor which has a molecular weight of approximately 94kD and which is processed within the cell to the desired antigen. In the case of P.69 the precursor is approximately 93.5kD. The DNA encoding it has been cloned and sequenced by Charles et al., (PNAS, 86, pp 3554-3558, (1989)). The precursor of the P.68 antigen of B.bronchiseptica is approximately 94kD and the precursor for P.70 of B.parapertussis is approximately 95kD.

Pichia microorganisms transformed with multiple copies of DNA for the expression of an antigenic fragment of a pertactin antigen are also encompassed by the invention. The fragments preferably contain no more than 50 amino acid residues. More preferably they contain between 5 and 25 residues. The fragments most preferably comprise a . defined antigenically effective sequence which essentially consists of amino acid residues 547 to 552 of the P.69 protein of B.pertussis.

This sequence is :

PGPQFP

The corresponding sequence for other strains of B.pertussis and for strains of B.parapertussis and B.bronchiseptica can be readily determined by lining up the amino acid sequence of the P.69 antigen, the P.70 antigen or the P.68 antigen respectively with the P.69 sequence shown by Charles et al (1989) referred to hereinbefore.

The fragments described above also include a sequence which essentially consists of the amino acid residues 544 to 566 of the P.69 protein of B.pertussis.

This sequence is :

APQPGPQPPQPPQPQPEAPAPQP

This sequence and the corresponding sequence for the P.70 antigen of B.parapertussis and the P.68 antigen of B.bronchiseptica can be aligned. A further fragment of interest is a 60 kD fragment encoded by the C terminal end of the DNA for P.69 which has been identified by Charles et. al. (1989) referred to hereinbefore as encoding an antigenic fragment of P.69.

Transformation of the organism may be carried out by any known method in the literature (Beggs, Nature 275, 104-109 (1978)). It is preferable to use the sphaeroplast method described by Cregg et al., Bio/Technology 5 479-485 (1987). The Pichia organism is preferably transformed with an expression cassette. Expression cassettes include DNA sequences in addition to that encoding the sequence of interest, in this instance the DNA encoding a pertactin antigen, such as transcriptional and translational initiation and termination sequences. The cassette may also include regulatory (i.e. promoter) and/or selectable marker sequences. Such expression cassettes are well known in the art and it is well within the ability of the skilled man to construct them. The expression cassette may form part of a vector construct or a naturally-occurring plasmid.

In a preferred embodiment the vector construct used to transform Pichia cells contains the promoter from the methanol-inducible A0X1 gene to drive expression of the DNA encoding a pertactin antigen. In particular, the vector may be pPIC3-60.5K. This vector, digested with BglII, will integrate in the host chromosomal A0X1 locus. The resultant aox1 transformants have the Mut^{s} (methanol-utilisation slow) phenotype and can therefore be selected.

In the invention, a Pichia organism is transformed with more than one copy of the DNA encoding a pertactin antigen or an antigenic fragment thereof. The DNA is integrated in the Pichia chromosomal DNA. It is preferable for the organism to contain greater than 5 copies preferably greater than 10 copies and most preferably between 5 and 30 copies of the DNA encoding pertactin antigen or an antigenic fragment thereof.

Such transformants can produce up to 5% of cell protein as the desired protein. In optimal fermenter conditions, levels of up to 10% of cell protein can be produced as the heterologous antigen. At such levels the majority of the antigen is insoluble. This is advantageous since the material can be readily isolated, renatured, and purified from the insoluble fraction.

The preferred Pichia organism referred to above is Pichia pastoris.

The invention further provides a procees for producing a pertactin antigen or an antigenic fragment thereof, which process comprises culturing a transformed Pichia microorganism of the present invention.

Culturing of the transformed microorganism is carried out by known methods in for example a yeast extract band medium using a shake flask. For optimal conditions it is preferable to use a fermenter equipped for monitoring pH, O₂, stir speed, temperature and or air flow, to control the cells' environment. The antigen produced by such methods can be isolated by centrifugation and purified by chromatography.

In order to obtain organisms containing more than one copy of the DNA encoding a pertactin antigen it is necessary to prepare a library of transformants using standard techniques for example as described by Cregg et al., Bio/Technology 5 : 479-485 (1987). Transformants containing at least one integrated copy of the DNA encoding a pertactin antigen form approximately 10-20% of these transformants. Those containing multiple copies of the DNA are then identified by screening. Individual transformants are grown on microtitre plates, each microculture is then transferred onto nitrocellulose filters and probed by Southern Hybridisation using pertactin-specific DNA radiolabelled to high specific activity using random-primed labelling (Feinberg et al., (1989) Anal. Biochem., 132- 6-13). A weak signal indicates that transformants contain a single copy of the DNA, a stronger signal indicates that the transformant contains more than one copy of DNA. The filters can then be mapped with the microtitre plates to identify the desired multi-copy microculture(s).

A Pichia organism according to the invention can therefore be produced by a process comprising :
i) transforming a Pichia organism with a vector contruct containing DNA encoding a pertactin antigen or a fragment thereof and a selectable marker,
ii) screening Pichia cells by means of selectable marker to select a transformed organism,
iii) screening said transformed organism to identify transformants containing said DNA or fragment thereof.

A process for the enhanced production of a pertactin antigen, or an antigenic fragment thereof, comprises :
a) transforming a Pichia organism with multiple vectors constructs containing DNA encoding a pertactin antigen or a fragment thereof operably linked to a promoter to drive expression of said DNA; and thereafter
b) culturing the resultant transformed organism under suitable conditions to obtain production of the antigen encoded by the DNA.

The present invention will now be exemplified further with reference to the accompanying figures.

### FIGURE LEGENDS

**Fig.1A.** The amino acid sequence of P.69 and the DNA sequence encoding P.69 from B.pertussis and its 93.5 kD precursor.

**Fig.1B.** The amino acid sequence of P.68 and the DNA sequence encoding P.68 from B.bronchiseptica and its 94 kD precursor.

**Fig.1C.** The amino acid sequence of P.70 and the DNA sequence encoding P.70 from B.parapertussis and its 95 kD precursor.

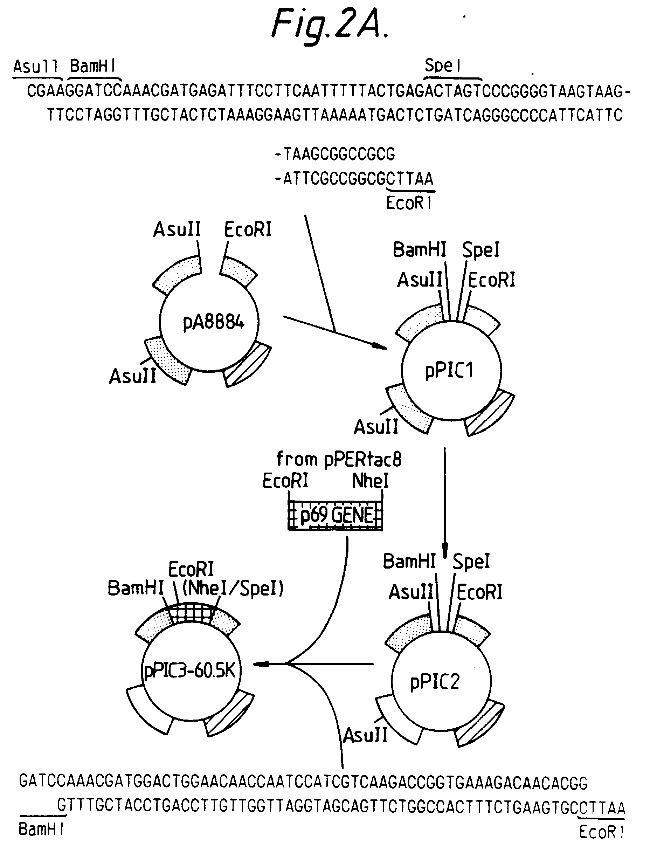

**Fig.2A.** Construction of the vector pPIC3-60.5K.

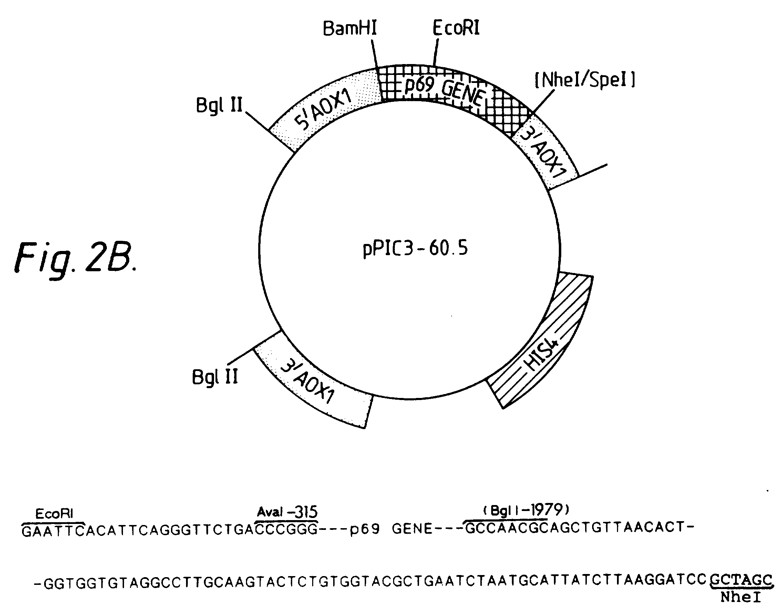

**Fig.2B** A plasmid map of the vector pPIC3-60.5K is shown in part A. Part B gives details of the DNA sequence of the DNA encoding P.69 that was used in the construction of pPIC3-60.5K. The EcoRI-NheI DNA fragment used contains the region from AvaI (nt.315) to BglI (nt.1979) from the 93kD precursor gene (Charles et al. (1989), PNAS 86, 3554-3558), flanked by sequences derived from the vector pPERtac8 (Makoff et al. Bio/Technology 8,1030 (1990)). This fragment was inserted into pPic2 using the adapter oligonucleotides shown, which encode the 5' end of the DNA encoding P.69 gene.

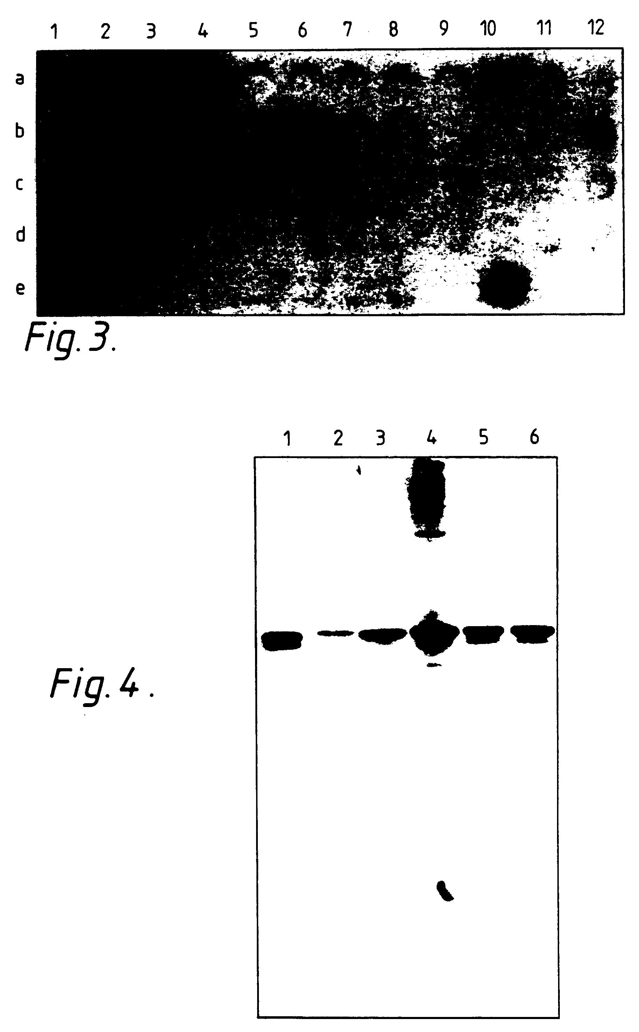

**Fig.3** DNA dot blot screen of GS115/pPIC3-60.5K Mut^{s} transformants. The filter shown had 56 transformants, a positive control (position E10: multi-copy transformant SL 22, identified in a previous screen), and a negative control (Ell: GS115). Most of the transformants in the screen gave a weak signal of similar intensity, a very small proportion gave a much stronger signal indicative of multi-copy integration (A3, A4, B6 : designated nos. SL3, SL4 and SL18). Four further filters were screened in the same way, but no more multi-copy transformants were found.

**Fig.4** Western blot analysis of P.69 pertactin induced P.pastoris cell extracts. P.pastoris transformants were induced for two days as described in Example 4. Proteins were separated by electrophoresis in 7.5% SDS-polyacrylamide gels and detected in Western blots using monoclonal antibody BB05 (Bio/Technology 8, 1030 (1990)). Tracks contained; authentic B.pertussis pertactin (1); or cell extracts from shake-flask induced; P.pastoris single-copy integrant SL1 (2), or from multi-copy integrant SL3 (3), SL4 (4), SL18 (5), and SL22 (6). Expression levels were estimated by comparison with known amounts of pertactin.

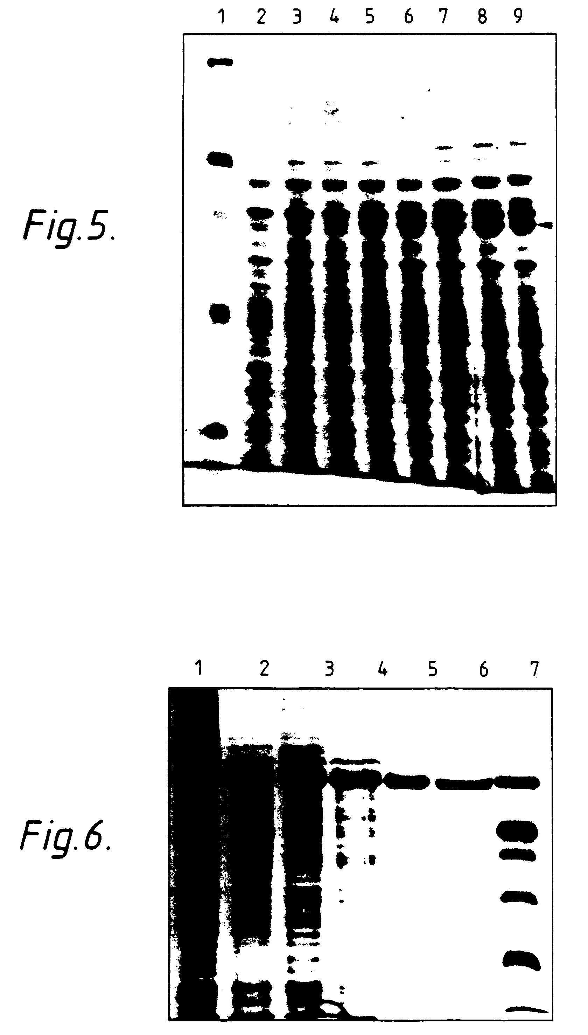

**Fig. 5** Analysis of pertactin expression from a SL22 fermenter induction. A fermenter protocol of batch growth in glycerol, followed by glycerol-starvation, then a controlled methanol feed was used as described in Example 5. Protein extracts (50µg) prepared from samples taken at different times after induction were analysed: tracks 2 to 9 correspond to -1, 0, 2, 4, 7, 23, 30, 50 hrs, respectively. Track 1 contained protein markers (Amersham Rainbow markers: myosin 200kDa, phosphorylase b 92.5kDa, bovine serum albumin 69kDa, ovalbumin 46kDa, carbonic anhydrase 30kDa). The induced pertactin band is indicated by an arrow. Densitometric scanning of the stained gel (Joyce-Loebl, Chromoscan) indicated that pertactin reached approximately 10% of cell protein after 30 hrs.

**Fig. 6** SDS-polyacrylamide gel analysis of pertactin purification. Induced SL4 cells were harvested, suspended in buffer A (50mM tris HCl pH8.0, 0.1M NaCl) + 1% triton X-100, and lysed with glass beads using a Bead Beater (Biospec Products, Bartesville, OK). Insoluble protein was pelletted by centrifugation, washed and suspended in 6M guanidinium thiocyanate/buffer A. On dialysis pertactin remained soluble while yeast proteins could be removed by centrifugation. Solubilised pertactin was loaded onto a chelating Sepharose column charged with zinc and equilibrated with buffer A. After washing with 0.5M NaCl, pertactin was eluted in 50mM MES pH6.0, 0.1M NaCl. Pertactin was further purified by chromatography on Q-sepharose in 50mM tris HCl pH8.0, with elution using a 0 to 0.4M NaCl gradient. Gel tracks : [1] 30µg total yeast protein [2] total soluble protein [3] guanidinium-solubilised fraction [4] Zn Sepharose-purified [5] Q-Sepharose-purified [6] native pertactin [7] Amersham Rainbow markers.

### EXAMPLES

General information pertinent to the examples:

### Media

YPD, 1 litre: 10g yeast extract, 20g peptone, 20g glucose. YNBBG, 1 litre: 13.4g yeast nitrogen base w/o amino acids (Difco LABs., Detroit, Michigan, USA), 0.4mg biotin, 20ml glycerol. YNBBGCas: same as above plus 10g casamino acids. YNBBD: same as YNBBG but 20g glucose instead of 20ml glycerol. YNBBM: same as YNBBG but 5ml methanol instead of 20ml glycerol. YNBBDCas and YNBBMCas had 10g casamino acids per litre. Solid media: as above plus 20g of agar per litre.

### EXAMPLE 1 - Construction of Pichia pastoris intracellular expression vectors for P.69

The vector pPIC3-60.5K, deposited at THE NATIONAL COLLECTION OF INDUSTRIAL AND MARINE BACTERIA LTD., P.O. Box 31, 135, Abbey Road, Aberdeen AB98DG, Scotland, UK on 30th March, 1990 under Accession No. 40270 derived from pAO804 (Digan et al, Dev.Ind.Microbiol. 1988 29, 59-65) was used for intracellular expression of P.69 in Pichia pastoris. This vector uses the promoter from the AOX1 gene to drive expression and can be integrated into the host chromosomal AOX1 locus. To facilitate insertion of the P.69 gene the synthetic oligonucleotides shown in Fig.2A were cloned between the AsuII and EcoRI sites of pAO804, to give pPICl. A derivative of this plasmid, pPIC2, which lacks the EcoRI site was then constructed. This was done by digesting with EcoRI followed by filling in of the protruding single stranded ends with the Klenow fragment of DNA polymerase I and ligating together the blunt ends. A 1.8kb EcoRI-NheI fragment (see Fig.2B) from the plasmid pPERtac8 (Makoff, et al, Bio/Technology 8, 1030 (1990)) which contains most of the gene encoding P.69 (a 60.5kD polypeptide) was inserted into BamHI-SpeI cut pPIC2, using the adapter oligonucleotides shown in Fig.2A, to give pPIC3-60.5K. These oligonucleotides encode the 5' end of the P.69 gene including the initiator ATG codon.

### EXAMPLE 2 - Transformation of Pichia pastoris

Pichia pastoris strain GS115 (his4⁻) was transformed with pPIC3-60.5K using the sphaeroplast method described by Cregg et al., Bio/Technology 5: 479-485 (1987). Transformants were regenerated on minimal medium lacking histidine, so that His⁺ colonies would be selected. The transforming DNA was 10 or 20ug of BglII-digested pPIC3-60.5K. This digest contains two DNA fragments, one of which (7.2 kb) has AOX1 sequences at either end, so that it is targeted to integrate at and replace ('transplace') the chromosomal AOX1 gene.

The His⁺ transformants generated are mainly found to contain undisrupted AOX1, and transplacements (aox1) may be isolated using a further screening procedure. Transplacements may be identified by their slow growth on methanol (Mut^{s} as opposed to Mut⁺ phenotype; Cregg et al., Bio/Technology 5 479-485, 1987). The transformants can be picked directly off regeneration plates and tested for growth on minimal methanol plates (YNBBM agar). Alternatively, the regeneration top agars can be lifted and homogenised in water, and the yeast cells plated to about 300 colonies per plate on minimal glucose plates (YNBBD agar). Mut^{s} colonies are then identified by replica-plating onto minimal methanol plates. In general, we and others have found the proportion of Mut^{s} to be 10-20% of all the transformants. Occasionally Mut⁺ transformants might be scored as Mut^{s} and these may also prove to contain multiple copies of the vector.

### EXAMPLE 3 - Screening for multi-copy transformants

In order to screen large numbers of transformants rapidly, they were grown in individual wells in a 96-place sterile microtitre plate. 200ul of YPD broth in each well was inoculated with a transformant, and the plates incubated for two days, at 30°C without shaking, to ensure growth to stationary phase. Included on each microtitre plate was a well containing GS115 (negative control) and one containing a known pPIC3-60.5K integrant (positive control). Using a multi-channel pipetter, 50ul samples of each micro-culture were transferred onto a nitrocellulose filter on a Schleicher & Schuell 'minifold' under vacuum. The filters were air dried, marked for orientation, then treated in the following way to lyse the cells: (i) 15min at room temperature with 50mM EDTA, 2.5% 2-mercaptoethanol pH9.0, (ii) 4hrs at 37°C with lmg/ml zymolyase (100T) in water, (iii) 5min at room temperature in 0.1M NaOH, 1.5M NaCl and (iv) twice for 5min at room temperature in 2 x SSC. Each treatment was performed by soaking 2 sheets of 3MM paper with the solution and placing the nitrocellulose filter on top. After these treatments the filters were baked at 80°C for 1hr.

The filters were probed by Southern hybridisation using P.69-specific DNA radiolabelled to high specific activity using random-primed labelling (Feinberg, A. and Vogelstein B., (1989), Anal. Biochem., 132, 6-13). Standard methods were used for prehybridisation, hybridisation, washing and autoradiography.

Fig.3 shows the results of such a screen of over 200 Mut^{s} transformants of pPIC3-60.5K. All the transformants reacted with the probe and most gave a similar weak signal (single-copy transformants), however, a very small proportion gave much stronger signals. These multi-copy transformants were tested further. The positive control in the filter shown is a transformant previously identified as multi-copy.

The copy number of the P.69 structural gene was determined by quantitative dot blot analysis of the DNA (Table 1). SL3, SL4 (Mut⁺), SL18 and SL22 gave significantly higher levels of product than single-copy integrants, up to 5% of cell protein (Table 1). The majority (90%) of the pertactin produced by SL4 and SL22 was insoluble. SL 22 was deposited at NCIMB, Aberdeen, Scotland under Accession Number 40391 on 22nd March 1991, in accordance with the terms of the Budapest Treaty.

### EXAMPLE 4 - Protein analysis

Selected transformants were cultured in YNBBGCas for 2 days at 30°C to reach stationary phase. These starter cultures were diluted to an OD₆₀₀ of 0.25 in 5ml fresh YNBBGCas and grown for 6hr. To induce these cultures the cells were collected by centrifugation, washed once in sterile water, and resuspended in YNBBMCas. Inductions were carried out for 2 days at 30°C.

Cells were then harvested by low speed centrifugation, washed once in water and suspended in 0.5 ml ice-cold break buffer (20mM sodium phosphate pH7.0, 0.1% triton X-100, 4mM phenylmethyl sulphonylfluoride). Acid washed glass beads (0.45mm) were added and the cells were broken by vigorous vortexing. The protein concentration of the extracts was determined using the BioRad protein assay (BioRad, according to manufacturer's instructions) and the material was stored at -20°C.

Proteins were separated by electrophoresis in 7.5% SDS-polyacrylamide gels (Laemmli U.K., Nature 227: 680-785, 1970). The proteins were visualised in the gel by staining with Coomassie Brilliant Blue R. Alternatively the proteins were transferred to a nitrocellulose filter and reacted with the P.69 specific monoclonal antibody BB05 (Montaraz et al., 1985, Infect. Immunity 47, 744-751; deposited at PHLS Public Health Laboratory Service Centre for Applied Microbiology and Research, Porton Down, Salisbury, Wiltshire, U.K. in the European collection of Animal cell cultures under Accession No. 90020103, on the 1st February, 1990 in accordance with the terms of the Budapest treaty), then with goat anti-mouse IgG conjugated to horse-radish peroxidase, and developed with H₂O₂ and 4-chloronaphthol (BioRad). In this way, the expressed P.69 could be specifically detected.

Fig.4 shows a Western blot of induced extracts of four selected multi-copy transformants (SL 3, 4, 18 and 22) and one typical single-copy transformant. By comparison to standard concentrations of pure P.69, it can be estimated that single-copy transplacements express P.69 at 0.1-0.5% of total cell proteins (t.c.p.), SL22 at approx. 2%, and SL4 at approx. 5%. These levels are for shake flask induction conditions.

### EXAMPLE 5 - Expression of P.69 during high cell density fermentation

In optimal fermenter inductions an improvement in protein yield may be observed. Thus SL22 has been induced in a controlled fermenter (Example 5) and expresses P.69 at about 10% t.c.p. SL4, which was subsequently determined to be Mut⁺ showed no change in expression levels in the fermenter. In low-expressing transformants the P.69 is largely soluble (approximately 55%), whereas at high expression levels it is mainly (approximately 90%) insoluble. This is preferable since the material can be readily isolated, renatured and purified from the insoluble fraction.

Production of P.69 by high cell density Pichia pastoris cultures was carried out using SL 22 in a 2L Braun fermenter equipped with monitors and controls for pH, dissolved O₂, stirring speed, temperature and air flow. A 10ml YNBBG overnight culture was used to inoculate the fermenter containing 1 litre of 5 x basal salts (phosphoric acid, 42mls/L; calcium sulphate 2H₂O, 1.8g/L; potassium sulphate 28.6g/L; magnesium sulphate 7H₂O, 23.4g/L; potassium hydroxide, 6.5g/L) with 4ml of PTM₁ salts (cupric sulphate 5H₂O, 6g/L; potassium iodide, 0.08g/L; manganese sulphate H₂O, 3g/L; sodium molybdate, 0.2g/L; ferrous sulphate 7H₂O, 65h/L; biotin, 0.2g/L; sulphuric acid, 5ml/L) and 5% (v/v) glycerol at 30°C. Dissolved oxygen was maintained above 20% by adjusting aeration and agitation, and the pH was maintained at pH5.0 by the addition of 50% (v/v) ammonium hydroxide. Growth was continued until the glycerol was exhausted (24-30hr). A limited glycerol feed (containing 50% w/v glycerol and 12ml/L PTM₁ salts) was then initiated at 12ml/hr for 17-21 hr. After this period the culture was induced by replacing the glycerol feed with a methanol feed (100% methanol plus 12ml/L PTM₁ salts) at 1ml/hr for 2hr. Then the methanol feed rate was gradually increased over a period of 6 hr to 6ml/hr and the fermentation was continued using these conditions for a further 40hr. At this point the methanol feed rate was reduced to 2ml/hr.

Samples were taken from the fermenter at different times after induction, and were analysed for P.69 expression as described in Example 4. The results of a Western blot analysis are shown in Fig.5, indicating that levels of P.69 of about 10% of cell protein were achieved.

### EXAMPLE 6 - Renaturation and purification of P.69

Induced SL4 cells were harvested, suspended in buffer A (50mM tris HCl pH8.0, 0.1M NaCl) + 1% triton X-100, and lysed with glass beads using a Bead Beater (Biospec Products, Bartesville, OK). Insoluble protein was pelletted by centrifugation, washed and suspended in 6M guanidinium thiocyanate/buffer A. On dialysis pertactin remained soluble while yeast proteins could be removed by centregufation. Solubilised pertactin was loaded onto a chelating Sepharose column charged with zinc and equilibrated with buffer A. After washing with 0.5M NaCl, pertactin was eluted in 50mM MES pH6.0, 0.1M NaCl. Pertactin was further purified by chromatography on Q-sepharose in 50mM tris HCl pH8.0, with elution using a 0 to 0.4M NaCl gradient. Overall recovery of pertactin was 40%.

### EXAMPLE 7 - Protection Data

Recombinant Pichia derived P.69 was tested for its ability to stimulate protection by toxoid in the Kendrick test, which is the standard international potency assay for whole-cell whooping cough vaccines.

Vaccines, containing 5µg (TEST A) OR 20µg (TEST B) of toxoid, 20µg P.69 and 10% alhydrogel/PBS, were serially diluted in PBS and 0.5ml doses were given intraperitoneally to male and female NIH/S mice.

Control mice received whole-cell vaccine (British ref. 66/84; top dose 8 IU/ml). After 14 days the mice were challenged intracerebrally with 20µl B.pertussis 18-323 (approximately 50LD₅₀).

Table 2 shows the results of two experiments, comparing toxoid with or without added P.69. Toxoid alone was clearly less protective than the whole-cell reference vaccine and could not be improved by increasing the dose from 5µg to 20µg. Addition of native or yeast-derived pertactin increased protection to the level of the whole-cell vaccine. For comparison, P.69 produced from E.coli (A.J. Makoff et al Bio/Technology 8, 1030 (1990)) was also included in the test and gave similar results. The results with pure recombinant P.69 from two different sources show that the immunogenic effect was due to P.69 itself, rather than contaminating B.pertussis antigens.

**Table 1.**

| Expression of P.69 pertactin in recombinant Pichia. | | | |
|---|---|---|---|
| Yeast/plasmid | Copy no.^{a} | Expression level ^{b} (% cell protein) | Solubility |
| pPIC3-P69 (SL1,Mut^{s}) | 1 | 0.5% | 50% |
| | | | |
| pPIC3-P69 (SL18,Mut^{s}) | 12 | <2% | nd |
| | | | |
| pPIC3-P69 (SL3,Mut^{s}) | 13 | <2% | nd |
| | | | |
| pPIC3-P69 (SL22,Mut^{s}) | 21 | 2% (10%^{C}) | <10% |
| | | | |
| pPIC3-P69 (SL4,Mut⁺) | 30 | 5% (6%^{C}) | <10% |

| | | | |
|---|---|---|---|
| ^{a} The integrated vector copy numbers were determined accurately by dot blot analysis of purified DNA probed with radiolabelled P.69 structural DNA or DNA from the single-copy P.pastoris ARG4 (supplied by K.Shreekrishna, Phillips Petroleum Co.). A transformant known to be single-copy from Southern analysis was used as a control. Hybridised probe was measured by scintillation counting. | | | |
| ^{b} Shake-flask inductions. | | | |
| ^{c} Peak levels in fermenter inductions. | | | |

**Table 2.**

| Survivors after challenge^{a} | | | | |
|---|---|---|---|---|
| Antigen dilution | 1 | 1/4 | 1/16 | 1/64 |
| TEST A | | | | |
| Toxoid alone | 10 | 2 | 0 | 1 |
| | | | | |
| Toxoid and native P.69 | 14 | 8 | 0 | 0 |
| | | | | |
| Toxoid and E.coli recombinant P.69 | 17 | 8 | 1 | 0 |
| | | | | |
| Reference vaccine | 15 | 7 | 1 | 1 |

| TEST B | | | | |
|---|---|---|---|---|
| Toxoid alone | 8 | 3^{b} | 1 | 0 |
| | | | | |
| Toxoid and native P.69 | 15 | 11 | 2 | 2 |
| | | | | |
| Toxoid and Pichia recombinant P.69 | 15 | 10 | 3 | 0 |
| | | | | |
| Reference vaccine | 15 | 7 | 2 | 1 |

| | | | | |
|---|---|---|---|---|
| ^{a} 18 animals per group | | | | |
| ^{b} 17 animals in this group | | | | |

## Claims (Claims for the following Contracting State(s): AT, BE, CH, DE, DK, FR, GB, GR, IT, LI, LU, NL, SE)

1. A *Pichia* microorganism transformed with multiple copies of DNA which encodes a pertactin antigen or an antigenic fragment thereof, the said multiple copies being integrated into the *Pichia* chromosomal DNA.

2. A *Pichia* microorganism as claimed in claim 1, wherein the said DNA encodes a pertactin antigen whose amino acid sequence is at least 98% homologous with that set forth in Figure 1A, 1B or 1C, or an antigenic fragment thereof.

3. A *Pichia* microorganism as claimed in claim 2, wherein the said DNA encodes a pertactin antigen whose amino acid sequence is substantially the same as that set forth in Figure 1A, 1B or 1C, or an antigenic fragment thereof.

4. A *Pichia* microorganism as claimed in claim 1, wherein the said DNA encodes the P69 antigen of *Bordetella pertussis.*

5. A *Pichia* microorganism as claimed in claim 1, wherein the said DNA encodes the P68 antigen of *Bordetella bronchiseptica.*

6. A *Pichia* microorganism as claimed in claim 1, wherein the said DNA encodes the P70 antigen of *Bordetella parapertussis.*

7. A *Pichia* microorganism as claimed in any one of claims 1 to 3, wherein the antigenic fragment essentially consists of the amino acid sequence: PGPQPP; or

APQPGPQPPQPPQPQPEAPAPQP

8. A *Pichia* microorganism as claimed in any one of the preceding claims, wherein between 5 and 30 copies of the said DNA are integrated into the *Pichia* chromosomal DNA.

9. A *Pichia* microorganism as claimed in any one of the preceding claims, which is *Pichia pastoris*.

10. A process for producing a pertactin antigen or antigenic fragment thereof, which process comprises culturing a transformed *Pichia* microorganism as defined in any one of the preceding claims.

## Claims (Claims for the following Contracting State(s): ES)

1. A process for producing a pertactin antigen or antigenic fragment thereof, which process comprises culturing a *Pichia* microorganism transformed with multiple copies of DNA which encodes a pertactin antigen or an antigenic fragment thereof, the said multiple copies being integrated into the *Pichia* chromosomal DNA.

2. A process as claimed in claim 1, wherein the said DNA encodes a pertactin antigen whose amino acid sequence is at least 98% homologous with that set forth in Figure 1A, 1B or 1C, or an antigenic fragment thereof.

3. A process as claimed in claim 2, wherein the said DNA encodes a pertactin antigen whose amino acid sequence is substantially the same as that set forth in Figure 1A, 1B or 1C, or an antigenic fragment thereof.

4. A process as claimed in claim 1, wherein the said DNA encodes the P69 antigen of *Bordetella pertussis.*

5. A process as claimed in claim 1, wherein the said DNA encodes the P68 antigen of *Bordetella bronchiseptica.*

6. A process as claimed in claim 1, wherein the said DNA encodes the P70 antigen of *Bordetella parapertussis.*

7. A process as claimed in any one of claims 1 to 3, wherein the antigenic fragment essentially consists of the amino acid sequence: PGPQPP; or

APQPGPQPPQPPQPQPEAPAPQP

8. A process as claimed in any one of the preceding claims, wherein between 5 and 30 copies of the said DNA are integrated into the *Pichia* chromosomal DNA.

9. A process as claimed in any one of the preceding claims, wherein the *Pichia* microorganism is *Pichia pastoris.*

10. A transfected *Pichia* microorganism as defined in any one of the preceding claims.

## Patentansprüche (Patentansprüche für folgende(n) Vertragsstaat(en): AT, BE, CH, DE, DK, FR, GB, GR, IT, LI, LU, NL, SE)

1. *Pichia*-Mikroorganismus, der mit mehreren Kopien einer DNA transformiert ist, die ein Pertactin-Antigen oder ein antigenes Fragment davon codiert, wobei die mehreren Kopien in die chromosomale *Pichia*-DNA integriert sind.

2. *Pichia*-Mikroorganismus gemäß Anspruch 1, wobei die DNA ein Pertactin-Antigen, dessen Aminosäuresequenz zu mindestens 98 % mit der in der Figur 1A, 1B oder 1C dargestellten homolog ist, oder ein antigenes Fragment davon codiert.

3. *Pichia*-Mikroorganismus gemäß Anspruch 2, wobei die DNA ein Pertactin-Antigen, dessen Aminosäuresequenz im wesentlichen die gleiche ist, wie jene, welche in der Figur 1A, 1B oder 1C dargestellt ist, oder ein antigenes Fragment davon codiert.

4. *Pichia*-Mikroorganismus gemäß Anspruch 1, wobei die DNA das P69-Antigen von *Bordetella pertussis* codiert.

5. *Pichia*-Mikroorganismus gemäß Anspruch 1, wobei die DNA das P68-Antigen von *Bordetella bronchiseptica* codiert.

6. *Pichia*-Mikroorganismus gemäß Anspruch 1, wobei die DNA das P70-Antigen von *Bordetella parapertussis* codiert.

7. *Pichia*-Mikroorganismus gemäß mindestens einem der Ansprüche 1 bis 3, wobei das antigene Fragment im wesentlichen besteht aus folgender Aminosäuresequenz: PGPQPP oder

APQPGPQPPQPPQPQPEAPAPQP .

8. *Pichia*-Mikroorganismus gemäß mindestens einem der vorhergehenden Ansprüche, wobei zwischen 5 und 30 Kopien der DNA in die chromosomale Pichia-DNA integriert sind.

9. *Pichia*-Mikroorganismus gemäß mindestens einem der vorhergehenden Ansprüche, welcher *Pichia pastoris* ist.

10. Verfahren zur Herstellung eines Pertactin-Antigens oder eines antigenen Fragments davon, wobei das Verfahren das Kultivieren eines transformierten *Pichia*-Mikroorganismus, wie er in mindestens einem der vorhergehenden Ansprüche definiert ist, umfaßt.

## Patentansprüche (Patentansprüche für folgende(n) Vertragsstaat(en): ES)

1. Verfahren zur Herstellung eines Pertactin-Antigens oder eines antigenen Fragmentes davon, wobei das Verfahren das Kultivieren eines *Pichia*-Mikroorganismus umfaßt, der mit mehreren Kopien einer DNA transformiert ist, die ein Pertactin-Antigen oder ein antigenes Fragment davon codiert, wobei die mehreren Kopien in die chromosomale *Pichia*-DNA integriert sind.

2. Verfahren gemäß Anspruch 1, wobei die DNA ein Pertactin-Antigen, dessen Aminosäuresequenz zu mindestens 98 % mit der in der Figur 1A, 1B oder 1C dargestellten homolog ist, oder ein antigenes Fragment davon codiert.

3. Verfahren gemäß Anspruch 2, wobei die DNA ein Pertactin-Antigen, dessen Aminosäuresequenz im wesentlichen die gleiche ist, wie jene, welche in der Figur 1A, 1B oder 1C dargestellt ist, oder ein antigenes Fragment davon codiert.

4. Verfahren gemäß Anspruch 1, wobei die DNA das P69-Antigen von *Bordetella pertussis* codiert.

5. Verfahren gemäß Anspruch 1, wobei die DNA das P68-Antigen von *Bordetella bronchiseptica* codiert.

6. Verfahren gemäß Anspruch 1, wobei die DNA das P70-Antigen von *Bordetella parapertussis* codiert.

7. Verfahren gemäß mindestens einem der Ansprüche 1 bis 3, wobei das antigene Fragment im wesentlichen besteht aus folgender Aminosäuresequenz: PGPQPP oder

APQPGPQPPQPPQPQPEAPAPQP .

8. Verfahren gemäß mindestens einem der vorhergehenden Ansprüche, wobei zwischen 5 und 30 Kopien der DNA in die chromosomale *Pichia*-DNA integriert sind.

9. Verfahren gemäß mindestens einem der vorhergehenden Ansprüche, wobei der *Pichia*-Mikroorganismus *Pichia pastoris* ist.

10. Transfizierter *Pichia*-Mikroorganismus, wie er in mindestens einem der vorhergehenden Ansprüche definiert ist.

## Revendications (Revendications pour l'(les) Etat(s) contractant(s) suivant(s): AT, BE, CH, DE, DK, FR, GB, GR, IT, LI, LU, NL, SE)

1. Micro-organisme *Pichia* transformé avec des copies multiples d'ADN qui code pour un antigène pertactine ou un fragment antigénique de celui-ci, lesdites multiples copies étant intégrées dans l'ADN chromosomique de *Pichia.*

2. Micro-organisme *Pichia* selon la revendication 1, dans lequel ledit ADN code pour un antigène pertactine dont la séquence d'acides aminés est au moins à 98 % homologue de celle décrite dans la Figure 1A, 1B ou 1C, ou un fragment antigénique de celui-ci.

3. Micro-organisme *Pichia* selon la revendication 2, dans lequel ledit ADN code pour un antigène pertactine dont la séquence d'acides aminés est substantiellement identique à celle décrite dans la Figure 1A, 1B ou 1C, ou un fragment antigénique de celui-ci.

4. Micro-organisme *Pichia* selon la revendication 1, dans lequel ledit ADN code pour l'antigène P69 de *Bordetella pertussis.*

5. Micro-organisme *Pichia* selon la revendication 1, dans lequel ledit ADN code pour l'antigène P68 de *Bordetella bronchiseptica.*

6. Micro-organisme *Pichia* selon la revendication 1, dans lequel ledit ADN code pour l'antigène P70 de *Bordetella parapertussis.*

7. Micro-organisme *Pichia* selon l'une quelconque des revendications 1 à 3, dans lequel le fragment antigénique consiste essentiellement en la séquence d'acides aminés : PGPQPP ; ou

APQPGPQPPQPPQPQPEAPAPQP

8. Micro-organisme *Pichia* selon l'une quelconque des revendications précédentes, dans lequel de 5 à 30 copies dudit ADN sont intégrées dans l'ADN chromosomique de *Pichia.*

9. Micro-organisme *Pichia* selon l'une quelconque des revendications précédentes, et qui est *Pichia pastoris.*

10. Procédé de production d'un antigène pertactine ou d'un fragment antigénique de celui-ci, procédé qui comprend la culture d'un micro-organisme *Pichia* transformé tel que défini dans l'une quelconque des revendications précédentes.

## Revendications (Revendications pour l'(les) Etat(s) contractant(s) suivant(s): ES)

1. Procédé de production d'un antigène pertactine ou d'un fragment antigénique de celui-ci, procédé qui comprend la culture d'un micro-organisme *Pichia* transformé avec des copies multiples d'ADN qui code pour un antigène pertactine ou un fragment antigénique de celui-ci, lesdites multiples copies étant intégrées dans l'ADN chromosomique de *Pichia.*

2. Procédé selon la revendication 1, dans lequel ledit ADN code pour un antigène pertactine dont la séquence d'acides aminés est au moins à 98% homologue de celle décrite dans la Figure 1A, 1B ou 1C, ou un fragment antigénique de celui-ci.

3. Procédé selon la revendication 2, dans lequel ledit ADN code pour un antigène pertactine dont la séquence d'acides aminés est substantiellement identique à celle décrite dans la Figure 1A, 1B ou 1C, ou un fragment antigénique de celui-ci.

4. Procédé selon la revendication 1, dans lequel ledit ADN code pour l'antigène P69 de *Bordetella pertussis.*

5. Procédé selon la revendication 1, dans lequel ledit ADN code pour l'antigène P68 de *Bordetella bronchiseptica.*

6. Procédé selon la revendication 1, dans lequel ledit ADN code pour l'antigène P70 de *Bordetella parapertussis.*

7. Procédé selon l'une quelconque des revendications 1 à 3, dans lequel le fragment antigénique consiste essentiellement en la séquence d'acides aminés : PGPQPP ; ou

APQPGPQPPQPPQPQPEAPAPQP

8. Procédé selon l'une quelconque des revendications précédentes, dans lequel de 5 à 30 copies dudit ADN sont intégrées dans l'ADN chromosomique de *Pichia.*

9. Procédé selon l'une quelconque des revendications précédentes, dans lequel le micro-organisme *Pichia* est *Pichia pastoris.*

10. Micro-organisme *Pichia* transfecté tel que défini dans l'une quelconque des revendications précéden_{tes.}
